# EUROPEAN PATENT SPECIFICATION

(11) **EP 2 587 940 B1**
(45) Date of publication and mention of the grant of the patent: **06.08.2014**
(21) Application number: 11725909.3
(22) Date of filing: 08.06.2011
(51) Int. Cl.: A23L 1/275, A23L 1/30, A23L 1/305, A61K 9/14, A61K 36/9066

(54) **PARTICLES COMPRISING HYDROPHOBIC POLYMER AND HYDROPHOBIC PHENOLIC COMPOUND**
PARTIKEL MIT EINEM HYDROPHOBEN POLYMER UND HYDROPHOBE PHENOLVERBINDUNG
PARTICULES COMPRENANT UN POLYMÈRE HYDROPHOBE ET UN COMPOSÉ PHÉNOLIQUE HYDROPHOBE

(30) Priority: 30.06.2010 EP 10167853
(43) Date of publication of application: 08.05.2013
(73) Proprietor: Unilever N.V., 3013 AL Rotterdam (NL); Unilever PLC, London, Greater London EC4P 0DY (GB)
(72) Inventor: PATEL, Ashok,Ranchodbhai, NL-3133 AT Vlaardingen (NL); TIWARI, Jyoti,Kumar, NL-3133 AT Vlaardingen (NL); VELIKOV, Krassimir, Petkov, NL-3133 AT Vlaardingen (NL)
(74) Representative: Tansley, Sally Elizabeth
(86) International application number: PCT/EP2011/059502
(87) International publication number: WO 2012/000757

(56) References cited:
- WO-A1-2006/120227
- WO-A2-90/03123
- US-A1- 2005 202 092
- US-A1- 2008 311 209
- US-A1- 2009 035 440
- BISHT SAVITA ET AL: "Polymeric nanoparticle-encapsulated curcumin (nanocurcumin): a novel strategy for human cancer therapy", JOURNAL OF NANOBIOTECHNOLOGY, BIOMED CENTRAL, LONDON, GB, vol. 5, no. 1, 17 April 2007 (2007-04-17), page 3, XP021024532, ISSN: 1477-3155, DOI: 10.1186/1477-3155-5-3
- SIEBERT K J ET AL: "Nature of polyphenol-protein interactions", JOURNAL OF AGRICULTURAL AND FOOD CHEMISTRY, AMERICAN CHEMICAL SOCIETY, US, vol. 44, no. 1, 1 January 1996 (1996-01-01), pages 80-85, XP002197460, ISSN: 0021-8561, DOI: DOI:10.1021/JF9502459
- LUCK G ET AL: "POLYPHENOLS, ASTRINGENCY AND PROLINE-RICH PROTEINS", PHYTOCHEMISTRY, PERGAMON PRESS, GB, vol. 37, no. 2, 1 January 1994 (1994-01-01), pages 357-371, XP001063990, ISSN: 0031-9422, DOI: DOI:10.1016/0031-9422(94)85061-5

## Description

The present invention relates to a composition in the form of particles comprising a hydrophobic polymer and a hydrophobic phenolic compound selected from the group existing of curcuminoids, theaflavins, isoflavones, and polymethoxylated flavones. Moreover, the present invention relates to a method to prepare such compositions in the form of particles.

### BACKGROUND OF THE INVENTION

Curcuminoids, theaflavins, isoflavones, and polymethoxylated flavones are hydrophobic water-insoluble phenolic compounds that may have various physiological effects upon consumption. Curcumin is a natural dietary ingredient, which is abundantly found in the popular Indian spice turmeric (a spice derived from the perennial herb *Curcuma longa* L.), which is a member of the ginger family (Zingiberaceae). Curcumin belongs to the curcuminoids; is brightly colored and may be used as a food coloring; is approved as a food additive, with E-number E100 in Europe. It has been found to have antioxidant, anti-inflammatory, and anti-cancer properties. Curcumin is a hydrophobic compound, and hence is difficult to disperse in aqueous systems, like aqueous food products. As a result the presence of large particle in the product can cause sedimentation, aggregation, sandiness and chalkiness. Several attempts have been made to improve the dispersility of curcumin in water, in order to be able to use it in a wider range of food products, especially beverages, to provide its beneficial properties to consumers upon consumption. Moreover due to its poor solubility in aqueous dispersions, several other disadvantages may occur, such as poor bioaccessibility and bioavailability and reduced stability of curcumin at intestinal pH (∼7.1) or alkaline conditions, due to hydrolysis of curcumin.

Also tea theaflavins show reduced stability of the theaflavins at intestinal pH (∼7.1) when theaflavins are present in dispersion. Moreover, disadvantages caused by poor solubility like sedimentation in beverages and low bio-availability upon ingestion and may occur. Similar disadvantages may arise for the hydrophobic isoflavones and polymethoxylated flavones. Several attempts have been made to overcome these disadvantages.

US 2002/026886 A1 discloses water dispersible compositions containing natural hydrophilic, water-insoluble pigments which are useful for the coating and colouring of edible products and pharmaceutical products. Example of such pigments are curcumin, carmine, and a carotenoid. The pigments may be present as bodies having a maximum size of 10 µm or smaller, down to 0.01 µm. The pigment can be mixed with a hydrophilic polymer, such as gelatin to coat edible products and pharmaceutical products.

WO 2006/022012 A1 discloses a solid dispersion of curcumin, which is soluble in an acidic solvent because of having an improved solubility of curcumin in water with the use of a solid dispersion technique. The curcumin composition is produced by dissolving curcumin together with polyvinylpyrrolidone in an alcoholic solvent and then spray-drying.

CN101433514A1 relates to curcumin poloxamer solid dispersion and a preparation method thereof. The preparation method comprises the following steps: weighing curcumin or derivative thereof and poloxamer 188 according to a mass ratio of between 1 to 3 and 1 to 20. Poloxamer 188 is a nonionic polyoxyethylene-polyoxypropylene block copolymer, used as surfactant. Curcumin is dispersed in the poloxamer in a molecular form or an unformed mode, so as to reach high dispersion state.

US 4,368,208 discloses a water-soluble curcumin complex suitable for use as a coloring agent in foods. This is prepared by dissolving and mixing a source of curcumin and gelatin in an aqueous acetic acid solution. The complex comprises up to about 15 percent curcumin by weight.

WO2006/120227 discloses an ingestible ink composition comprising a zein binder, a solvent and an ingestible pigment or dye, e.g. curcumin. The zein binder has to be soluble in the solvent and bind the pigment to a substrate upon drying of the ink. The dissolved zein may stabilise dispersions of pigment particles smaller than 0.4 µm.

WO 2009/101263 A2 discloses a composition comprising a water-soluble and stable complex formed by an alkyl ether derivative of gamma-cyclodextrin and curcumin and optionally comprising non-complexed cyclodextrin, the molar ratio of curcumin to cyclodextrin being between 1:1 and 1:6, and a method of manufacturing such a composition. The water-soluble and stable complex of curcumin is useful in therapy.

US 2006/067998 A1 discloses a colloidal drug delivery system of curcumin for parenteral use with main focus on liposomal formulation. The drug delivery system is lipid-based, and preferably a liposomal formulation. It is used in treatment of cancer in humans by parenteral administration (i.e. not oral).

WO 90/03123 discloses zein microparticles and their method of preparation. The colloidal particle can be used as replacement for fat droplets in foods. Particle size between about 0.5 and 4 micron have been reported.

WO 91/06286 discloses zein microspheres that can be used as carriers for compounds for subsequent release. These compounds can be biologically active compounds such as pharmaceuticals. Examples show particles having a diameter of 30-35 micrometers.

WO 2009/137112 A1 discloses a method of preparing zein nanoparticles using a pH precipitation method, leading to controlled particle size distribution from about 100 to 400 nanometer. Nanoconjugates can be made for use in a therapeutic method. The nanoparticles may be loaded by compounds, such as 6,7-hydroxy-coumarin.

A problem known in the art is that polyphenols are often found to provide unwanted astringency and bitterness to food compositions and may unwanted colouring to some products. To solve this problem, WO 2009/016018 (A1) discloses that complexation of polyphenols with a polymer comprising amine groups allows incorporation of polyphenols in products at high levels with minimum impact on product sensory properties and/or increased stability of the polyphenol in the product. The polyphenol is preferably present as part of a complex with the polymer. The complex is present in the composition in the form of particles. A method of manufacturing the complexes is disclosed, in which the complex is formed by contacting the polymer and polyphenol, in a supporting medium, for example in an aqueous supporting medium.

In general the methods used in the prior art patents result in formation of particles with larger micron sizes and wide particle size distribution. Traditionally, larger particles are not very stable in a liquid formulation leading to sedimentation over a period of time. Moreover disadvantage of large particles are sedimentation, aggregation, sandiness and chalkiness in food products upon consumption.

In general, improving solubility of the hydrophobic phenolic compounds alone is not sufficient to increase the low oral bioavailability. For example, curcumin is unstable under intestinal conditions (pH of about 7.1), due to rapid hydrolysis of curcumin at neutral and alkaline pH. Also theaflavins may show instability at about neutral pH. Hence the hydrophobic phenolic compounds should not only show improved solubility under aqueous conditions, but also exhibit improved stability under gastric conditions and show improved absorption from the gastro-intestinal lumen, in order to be an ingredient which can be efficiently and effectively used in food products.

### SUMMARY OF THE INVENTION

In spite of the advances shown in the prior art, there still is a need for improved systems containing hydrophobic phenolic compounds, wherein these systems show one or more of the following advantages:
- enhanced water dispersibility and stability against aggregation and sedimentation;
- enhanced pH stability and photo stability, e.g. discolouration;
- stability under digestive conditions;
- enhanced mucoadhesion properties;
- enhanced stability in aqueous food products, e.g. stability against possible adverse changes in taste.

Moreover, there still is a need for improved methods for preparing systems like the above. For instance, it generally is a problem to prepare such systems in which hydrophobic phenolic compounds are stably dispersed in, for example, water. In particular, methods known in the art are generally limited with respect to scope of the phenolic compounds and additional compounds in terms of their hydrophobicity or hydrophilicity. Another general problem is a need for improving for instance control over the size and morphology of such systems containing hydrophobic phenolic compounds.

It is therefore an object of the present invention to provide a system containing hydrophobic phenolic compounds with enhanced stability for use in food products.

It is a particular object of the invention to provide a system which enhances the water-dispersibility and colloidal stability (i.e. stability against aggregation and sedimentation) of the hydrophobic phenolic compounds it comprises.

It is another particular object of the invention to provide a system which enhances the pH stability and photostability of the hydrophobic phenolic compounds it comprises.

It is yet another particular object of the invention to provide a system which enhances the stability under digestive conditions to the hydrophobic compounds it comprises.

It is still another particular object of the invention to provide a system which enhances the mucoadhesion properties of the hydrophobic phenolic compounds it comprises.

It also is an object of the present invention to provide a method to prepare systems containing hydrophobic phenolic compounds with the above advantages.

We have now found that one or more of these objects can be met by particles comprising one or more prolamines, selected from the group existing of gliadin, hordein, secalin, zein, avenin, and combinations of these prolamines, and a hydrophobic phenolic compound, selected from the group existing of curcumin, theaflavins, isoflavones, and polymethoxylated flavones, and combinations of these phenolic compounds. We particularly found that these particles enhance the water-dispersibility, pH stability, stability under digestive conditions and the muco-adhesive properties of the hydrophobic phenolic compounds.

Moreover, we have now found that a method comprising the steps of dissolving a hydrophobic polymer and a hydrophobic phenolic compound in a mixture of water and organic solvent, wherein the solvent is miscible with water, and adding the resulting mixture to water under stirring can be used to obtain the abovementioned compositions in the form of a particle containing hydrophobic phenolic compounds.

Accordingly in a first aspect the present invention provides a composition in the form of a particle comprising a hydrophobic polymer
wherein the polymer comprises one or more prolamines, selected from the group existing of zein, gliadin, hordein, secalin, avenin, and combinations of these prolamines,
and a hydrophobic phenolic compound,
selected from the group existing of curcuminoids, theaflavins, isoflavones, and polymethoxylated flavones, and combinations of these phenolic compounds,
and wherein the particles have a volume weighted mean diameter of between 10 and 1000 nanometer.

In a second aspect the present invention provides a method for preparation of a composition in the form of a particle according to the first aspect of the invention, comprising the steps:
a) dissolve hydrophobic polymer and hydrophobic phenolic compound, selected from the group existing of curcuminoids, theaflavins, isoflavones, and polymethoxylated flavones, and combinations of these phenolic compounds, in a mixture of water and organic solvent, wherein the solvent is miscible with water;
b) add the mixture from step a) to water under stirring;
c) separate particles precipitated in step b) from the remaining liquid;
d) optionally dry the particles to remove water and solvent.

### DETAILED DESCRIPTION

Unless defined otherwise, all technical and scientific terms used herein have the same meaning as commonly understood by one of ordinary skill in the art.
All percentages, unless otherwise stated, refer to the percentage by weight.
In the context of the present invention, the average particle diameter is expressed as the d_{4,3} value, which is the volume weighted mean diameter, unless stated otherwise. The volume based particle size equals the diameter of a sphere that has same the same volume as a given particle.
The ranges that are indicated include the endpoints, unless stated otherwise, and are understood by the skilled person to be values which may vary within limits which are acceptable to the skilled person. These variations within certain limits may for instance be determined by measurement uncertainties.

### Hydrophobic phenolic compounds: curcumin, theaflavins, isoflavones, and polymethoxylated flavone

Hydrophobic in the context of the present invention indicates that the compound is practically not soluble in water (less than 0.1 milligram per milliliter water, at ambient temperature, 20-25°C), or slightly soluble in water (0.1 to 1 milligram per milliliter water at ambient temperature, 20-25°C).

Curcumin (diferuloylmethane) together with desmethoxycurcumin, and bis-desmethoxycurcumin forms the curcuminoid component obtained from the traditional Indian spice turmeric (*Curcuma longa*). The curcuminoids are phenolic compounds and are responsible for the yellow color of turmeric. Curcumin can exist in at least two tautomeric forms, keto and enol. The enol form is energetically more stable in the solid phase and in solution.

Curcumin keto form:

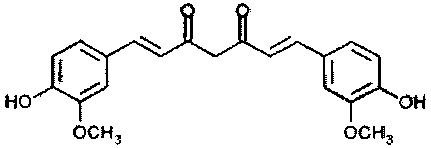

Curcumin enol form:

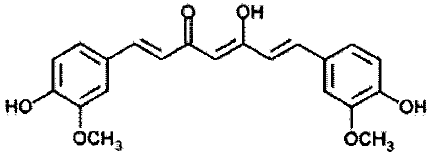

Molecular formula: C₂₁H₂₀O_{6;} molar mass 368.38 g/mol; bright yellow-orange powder; melting point 183°C.

Theaflavins is a collective term for theaflavin and its derivatives like theaflavin-3'-monogallate, theaflavin-3-gallate, theaflavin- 3'-gallate, theaflavin-3,3'-digallate and 3-isotheaflavin-3-gallate. Theaflavins (about 1-2% of the total dry matter of black tea) possess benzotropolone rings with dihydroxy- or trihydroxy-substitution. The theaflavins, are typically formed via polymerization of green tea polyphenols (i.e. flavanoids) during fermentation of green tea to black tea. Some of its physiological effects may include cholesterol reduction, colon health, cardiovascular health, heart disease prevention, immune support, anti-oxidation, and weight-loss (Maron D.J. et. al., 2003, Arch, Intern. Med. 163, 1448-1453; Friedman M., 2007, Mol. Nutr. Food Res. 51, 116-134). Literature suggests low solubility of these compounds in cold water which in turn results in cloudiness and creaming of tea on cooling (Jobstl E. et. al., 2005, J. Agri. Food Chem. 53, 7997-8002).

Isoflavones are phenolic compounds which are among others capable of exerting estrogen like effects. Examples of isoflavones are genistein, daidzein, formononetin and coumestrol. Interest for these substances originated from epidemiological findings indicating that they may provide protection against chronic diseases, such as hormone-dependent cancers and disorders of the cardiovascular system. Subsequent studies revealed that genistein, daidzein exhibit multiple pharmacological effects, with the result that at present the isoflavones are emerging as active ingredients for nutraceuticals or as prospective drug candidates. However, poor solubility in water along with rapid invivo metabolism results in low bioavailaibility of these compounds (Stancanelli R. et. al., 2007, J. Pharm. Biomed. Anal. 44, 980-984).

Polymethoxylated flavones, and also citrus flavonoids, are a group of low molecular weight polyphenol compounds with a broad range of biological activities. They are naturally found in the heavily pigmented yellow, red, and purple fruits, vegetables, teas, and wines as well as nuts, and seeds. Many of the nutritional actions of foods are directly related to their flavonoid content. For purposes of the present invention examples of naturally occurring polymethoxyflavones include, but are not limited to, hesperidin, naringin, tangeretin and nobiletin. In addition to acting as free-radical scavengers, they may exhibit anti-inflammatory properties or prevent/slow the development of some cancers. Kurowska E.M. et al (2004, J. Agric. Food Chem. 52, 2879-2886) has shown that polymethoxylated flavones are novel flavonoids with cholesterol- and triacylglycerol-lowering potential. Whitman S.C. et al. (2005, Atherosclerosis 178, 25-32) showed that nobiletin, a citrus flavonoid isolated from tangerines, inhibited (50-72%) acetylated LDL metabolism. Research performed by Lee C.H. et. al. (2001, Biochem Biophys Res Commun. 284, 681-688), suggest that the anti-atherogenic effect of citrus flavonoids, naringin and naringenin, is involved with a decreased hepatic ACAT activity. However, their physiological effects are often limited due to low oral bioavailability attributed to the low bioavailaibility and accordingly attempts have been made to improve their solubility (Yao J.J. et al., 2008, Pharm. Pharmaceut. Sci. 11, 22-29).

### Proteins - prolamines

Prolamines are a group of plant storage proteins having a high proline content and are found in the seeds of cereal grains. Examples of these grains are wheat (protein gliadin), barley (protein hordein), rye (protein secalin), corn (protein zein) and as a minor protein, avenin in oats. The prolamines are characterised by a high glutamine and proline content and are generally soluble only in strong alcohol solutions.

Zein is the alcohol-soluble protein of corn and is classified as a prolamin. Biologically, zein is a mixture of proteins varying in molecular size and solubility. These proteins can be separated by differential solubilities and their related structures into four distinct types: alpha (α), beta (β), gamma (γ), and delta (δ) zein. Alpha-Zein is by far the most abundant, accounting for about 70% of the total. The next most abundant zein is gamma-zein, contributing to about 20% of the total.

Gluten is a storage protein from wheat and comprises two major protein groups, namely the gliadins (molecular weight 30,000-80,000) and glutenin polymers (molecular weight higher than 100,000). It is classified as prolamines due to the presence of aqueous alcohol soluble gliadin groups.

Gliadin is a glycoprotein present in wheat and several other cereals within the grass genus Triticum. Gliadins are prolamins and are separated on the basis of electrophoretic mobility and isoelectric focusing. Together with glutenin it forms an important component of wheat gluten. Hordein is a major storage protein from barley. Its a glycoprotein also classified as prolamin based on its solubility characteristics. Secalin, a storage protein found in rye, with high glutamine and proline content and low lysine content is also classified as prolamin.

### Particles comprising hydrophobic polymer and hydrophobic phenolic compound

In a first aspect the present invention provides a composition in the form of a particle comprising a hydrophobic polymer
wherein the polymer comprises one or more prolamines, selected from the group existing of zein, gliadin, hordein, secalin, avenin, and combinations of these prolamines,
and a hydrophobic phenolic compound,
selected from the group existing of curcuminoids, theaflavins, isoflavones, and polymethoxylated flavones, and combinations of these phenolic compounds,
and wherein the particles have a volume weighted mean diameter of between 10 and 1000 nanometer. Preferably the particles have a volume weighted mean diameter between 20 and 800 nanometer, more preferably between 50 and 500 nanometer, more preferably between 60 and 300 nanometer. Most preferably the volume weighted mean diameter of the particles is between 80 and 250 nanometer. A smaller size of a nanoparticle generally leads to a faster dissolution rate, for example in gastro-intestinal fluid. For some purposes a small particle size may be required, for some other application a somewhat larger particle size may be better. Without being limited by theory, it is thought that by the preparation of the particles containing a prolamin, the bioadhesion of the particles to the gastrointestinal wall is improved, leading to improved transport of the phenolic compound across the gastrointestinal wall, as compared to the phenolic compound without the presence of a prolamin in a nanoparticle.

Preferably, the particles contain between 1 and 25% by weight of phenolic compound, based on the weight of the particle. More preferably between 1 and 23% by weight, more preferably between 2 and 20% by weight, more preferably between 3 and 18% by weight, more preferably between 4 and 16% by weight. If the ratio of phenolic compound becomes too high, the particles may become too big and be prone to sedimentation. Moreover the presence of a prolamine may improve the adhesion to the intestinal wall, and hence may promote bioavailability of the phenolic compound by improved transport across the intestinal wall. Most preferred the particles contain between 6 and 15% by weight of phenolic compound. If the relative amount of phenolic compound in a particle would become too low, then possibly the protein load in a product may become too high in order to deliver a suitable amount of phenolic compound.

Preferably, the composition in the form of a particle according to the present invention comprises prolamines, selected from the group existing of zein, gliadin, hordein, secalin, avenin, wherein the prolamines are very slightly soluble, more preferably practically insoluble in water. Preferably, the composition in the form of a particle according to the present invention comprises a hydrophobic phenolic compound, selected from the group existing of curcuminoids, theaflavins, isoflavones, and polymethoxylated flavones, and wherein the phenolic compound is very slightly soluble, more preferably practically insoluble in water. Preferably the composition in the form a particle according to the present invention is water-dispersable.

A compound is considered very slightly soluble if its solubility amounts to 100 to 1000 micrograms per millilitre and practically insoluble if its solubility is less than 100 micrograms per millilitre.

In addition to the prolamine and the phenolic compound, the particles according to the invention may also contain other stabilisers like surfactants, emulsifiers, surface active polymers, and other proteins to provide additional stabilisation benefit.

Without wishing to be bound by theory, it is believed that the morphology of the particles according to the present invention positively contributes to the enhanced water-dispersibility, pH stability, stability under digestive conditions and the muco-adhesive properties of the hydrophobic phenolic compounds. Here, morphology is understood as including aspects of the particle shape and size, as well as the internal distribution and mutual interactions of the prolamines and phenolic compounds within the particles. Such morphological features may for instance be controlled by the method by which the particles are prepared. Therefore, the particles according to the invention are preferably obtainable by the method according to the second aspect of the invention.

The morphology of the particles according to the present invention is preferably substantially convex and compact. More preferably their morphology is ellipsoidal, even more preferably it is spheroidal, and still more preferably it is substantially spherical. An ellipsoidal particle according to the invention preferably has an aspect ratio of smaller than 10 to 1. A particle is considered substantially spherical if its largest and its smallest cross-sectional diameter differ by less than 25%.

The particles according to the invention are generally well-dispersible in water: Preferably, at least 75 wt-%, more preferably at least 85 wt-% and still more preferably at least 95 wt-% of the phenolic compound comprised in a colloidal dispersion of particles according to the invention is dispersed within 5 minutes upon dissolution in a dissolution medium consisting of deionised water with 0.05% of polysorbate 80, using a USP 2 dissolution test.

The particles according to the invention generally show good pH stability. Preferably, at least 50 wt-%, more preferably at least 60 wt-% and even more preferably at least 70 wt-% of the phenolic compound comprised in the particles according to the invention is not degraded within six hours after dilution of a colloidal suspension of the particles in a buffer at a pH between 1.2 and 9.0 and at a phenolic compound concentration equivalent to 20 microgram per millilitre.

The particles according to the invention generally show good photostability. Preferably, at least 40 wt-%, more preferably at least 50 wt-% and even more preferably at least 60 wt-% of the phenolic compound comprised in the particles according to the invention is not degraded within 45 minutes, upon irradiation of a colloidal suspension of the particles at a concentration of the phenolic compound equivalent to 20 microgram per millilitre with UV light at a wavelength of 302 nm using a Bio-Rad Gel Doc 1000 minitransilluminator.

The particles according to the invention generally show good stability in digestive conditions, in the sense that they are not broken down by gastric and intestinal fluids. Therefore, the particles are suitable as a delivery system for phenolic compounds to the human body. Preferably, at least 50 wt-%, more preferably at least 60 wt-% and even more preferaby at least 70 wt-% of the phenolic compound comprised in the particles according to the invention remains in the dispersion for at least 3 hours following dissolution of a colloidal dispersion of the particles in a simulated gastro-intestinal medium, wherein the medium initially comprises 0.05 wt-% of polysorbate surfactant and pepsin and has a pH of 2, and wherein after 2 hours the pH is adjusted to 6.8 and bile salts and pancreatin are added.

The particles according to the invention generally show good muco-adhesive properties. Preferably at least 40 wt-%, more preferably at least 50 wt-% and even more preferably at least 60 wt-% of the particles adheres to mucin-coated CaCo2 cells upon incubation at 37°C for 2 hours.

### Compositions comprising the particles

Typically the particles according to the invention will be dispersed in a supporting medium. Preferably the supporting medium will make up the bulk of the composition and determine, to a large extent, its sensory and physical characteristics. The supporting medium may be any suitable substance and will depend to a large extent on the intended end use of the composition. Typically, however, the supporting medium will be a liquid, dispersion (single or duplex emulsion, foam or suspension), gel, solid, or may be a mixture thereof. The supporting medium may be aqueous (preferably comprising at least 50% water by weight of the supporting medium) or non-aqueous. In a most preferred embodiment, the properties of the particles, especially their size and surface properties, and those of the supporting medium, especially its viscosity and polarity, are selected such that the particles form a stable colloidal dispersion in the supporting medium. The dispersion is preferably stable such that no appreciable sedimentation of the particles occurs over a period of at least 7 days at a storage temperature of 20°C, more preferably over a period of at least 1 month and most preferably at least 6 months. Hence in a further aspect the present invention provides a composition comprising a composition in the form of a particle according to the first aspect of the invention, wherein the composition is in liquid, solid, or semi-solid form.

In a solid composition the particles of the present invention will generally be incorporated as a dry powder. In a liquid composition, the particles will generally be dispersed in a liquid. In a semi-solid or semi-liquid composition like gels, jellies or paste, particles will preferably be incorporated as aqueous dispersion.

Non-limiting examples of solid compositions are a nutritional bar and an instant powder for preparing a beverage. Non-limiting examples of liquid compositions are beverages. Non-limiting examples of semi-solid or semi-liquid compositions are spreads and yoghurt.

Preferably a composition comprising the particles according to the invention is edible. The edible composition preferably is a food product, as this allows for convenient and enjoyable consumption of the phenolic compound. The food composition may be, for example, a margarine, low fat spread, confectionery product (such as chocolate or cereal bar), ice cream, dressing, mayonnaise, sauce, bakery product, shortening or cheese. However, it is especially preferred that the food composition is a beverage. When the food composition is a beverage, it is preferably a coffee-based beverage, a tea-based beverage and/or a cocoa-based beverage. Most preferably the beverage is tea-based. The pH of the beverage may, for example, be from 2.5 to 8, preferably 3 to 6, more preferably from 3.5 to 5.

The food may be dried and contain less than 40% water by weight of the composition, preferably less than 25%, more preferably from 1 to 15%. Alternatively, the food may be substantially aqueous and contain at least 40% water by weight of the composition, preferably at least 50%, more preferably from 65 to 99.9%. The food preferably comprises nutrients including carbohydrate (including sugars and/or starches), protein, fat, vitamins, minerals, phytonutrients (including terpenes, organosulfides or a mixture thereof) or mixtures thereof. The food may be low calorie (e.g. have an energy content of less than 100 kCal per 100 g of the composition) or may have a high calorie content (e.g. have an energy content of more than 100 kCal per 100 g of the composition, preferably between 150 and 1000 kCal). The food may also contain salt, flavours, colours, preservatives, antioxidants, non-nutritive sweetener or a mixture thereof.

Moreover the composition according to the invention may be used in pharmaceutical products or in dietary supplements. The form of the composition may, among others, be a tablet, pill, lozenge, paste, lotion, gel, cream, liquid (including emulsion and/or suspension), spray (including aerosol spray), foam or powder. The pharmaceutical of the present invention may be suitable for any form of administration, preferably oral administration. The pharmaceutical composition generally will comprise a pharmaceutically acceptable vehicle which may act as a diluent, dispersant or carrier for the particles according to the invention. The vehicle may be aqueous or anhydrous.

### Method for preparation of particles

In a second aspect the present invention provides a method for preparation of a composition in the form of a particle according to the first aspect of the invention, comprising the steps:
a) dissolve hydrophobic polymer and hydrophobic phenolic compound, selected from the group existing of curcuminoids, theaflavins, isoflavones, and polymethoxylated flavones, and combinations of these phenolic compounds, in a mixture of water and organic solvent, wherein the solvent is miscible with water;
b) add the mixture from step a) to water under stirring;
c) separate particles precipitated in step b) from the remaining liquid;
d) optionally dry the particles to remove water and solvent.

Any organic solvent may be suitable, as long as the prolamine and the phenolic compound are soluble in the solvent. Preferably the ratio of organic solvent to water in step a) is 60:40 (v/v) or higher. In step a) preferably the mixture of water and organic solvent comprises ethanol, preferably at a ratio of ethanol to water of between 60:40 (v/v) and 90:10 (v/v). More preferred the ratio of ethanol to water is between 75:25 (v/v) and 85:15 (v/v).

In step b) the mixture from step a) is mixed with water under stirring. The type of stirrer and stirring speed are not critical, stirring is required to prevent localised precipitation of prolamine and/or polyphenol.

In step c) the precipitated particles are separated from the liquid by any suitable method, such as decanting, filtration, centrifugation, evaporation of the liquid phase or any other suitable method.

In optional step d) the particles are dried, to remove water and solvent. Drying can be performed by any suitable method, such as air drying and freeze drying.

Optionally, other stabilisers such as surfactants, emulsifiers, surface active (bio)polymers, and proteins (other than prolamines).can be included in the water phase or if soluble in the organic solvent containing phase.

An advantage of the method for the preparation of a composition in the form of a particle according to the present invention is that it is especially suitable for controlling the morphology of the particles. In particular the density and the shape of the particles may be controlled, leading for example to the formation of particles the morphology of which is substantially convex and compact. The method may even yield ellipsoidal, spheroidal, or substantially spherical particles.

Another advantage of the method for the preparation of a composition in the form of a particle according to the present invention is that it is especially suitable for the preparation of water-dispersable particles comprising a hydrophic phenolic compound and a hydrophobic polymer, even if one or more of the phenolic compounds or polymers themselves are not water-soluble.

The dried particles can be used further in formulating compositions in which the particles are incorporated, preferably food products. The dried particles do not need to be further formulated into a product, they could also be packed as such before being marketed.

Food products containing the particles according to the invention can be produced by any normal method for producing the food. For example the particles can be added in liquid and semi-liquid formats in form of powder or (concentrated) dispersion. In dry products, dried particles according to the invention can be mixed in or spray on to powder (granulation).

### Use of the particles

In a third aspect the present invention provides use of a composition in the form of a particle according to the invention for improving the muco-adhesive properties of hydrophobic phenolic compounds. Another use preferably relates to use for improving the stability of hydrophobic phenolic compounds in aqueous food products. Here, stability refers to one or more aspects of stability, selected from colloidal stability and chemical stability, more in particular the stability at different pH levels, under UV-light, or under gastro-intestinal conditions.

As indicated before, the phenolic compounds should not only show improved solubility under aqueous conditions, but also exhibit improved stability under gastric conditions and show improved absorption from the gastro-intestinal lumen. Improved stability can be measured by measuring the quantity of intact (not degraded) phenolic compound as a function of time. By the improved stability and dispersibility, transport of the phenolic compound across the intestinal wall is better facilitated, and therewith improved uptake of the phenolic compound can be achieved. This leads to higher bioavailability and consequently the beneficial properties of the phenolic compounds related to the health of the consumer can be better exploited. Therefore, use of the particles according to the present invention preferably also relates to use for improving the bioavailability of hydrophobic phenolic compounds.

The bioavailability of a compound is generally believed to be improved by good muco-adhesive properties. Therefore, use of the particles according to the present invention preferably also relates to use for improving the muco-adhesive properties of hydrophobic phenolic compounds.

### DESCRIPTION OF FIGURES

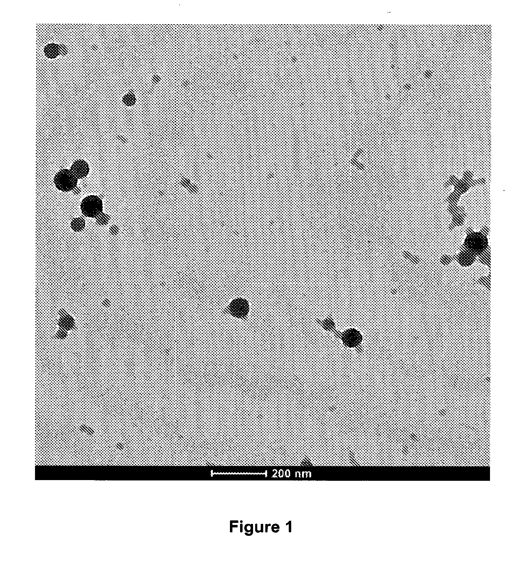

**Figure 1** is a transmission electron micrograph of zein/curcumin particles according to Example 1.7.

### EXAMPLES

The following non-limiting examples illustrate the present invention.

### Description of materials

Curcumin: Curcumin (95% purity as per supplier's claim) (ex Sanjivani Phytopharma Pvt. Ltd., India)
Theaflavin: was prepared from black tea extract by concentrating, i.e. removal of water through vacuum evaporation and drying. A light brown powder was obtained, and purity of theaflavin was determined (using HPLC and NMR) to be about 50%. The balance 50% of the material contains carbohydrates, proteins and oligomerized catechins and theaflavins.
Zein: Zein from maize (ex Sigma Aldrich Inc.)
Gluten: Gluten from wheat (Sigma Aldrich Inc.)
Tween 80: Emulsifier, synthesis grade (ex Merck Co.)
Pepsin: Pepsin from porcine gastric mucosa (ex Sigma Life Sciences)
Bile salts: Bile extract porcine (ex Sigma Life Sciences)
Pancreatin: Pancreatin porcine pancreas (ex Sigma Life Sciences)
Agarose: Agarose (ex Invitrogen Life technologies)
Simulated Gastric Fluid: prepared by diluting concentrated hydrochloric acid
CaCo2 cells: The Caco-2 cell line is a continuous line of heterogeneous human epithelial colorectal adenocarcinoma cells. They are generally used as monolayers of cells on cell culture insert filter) They were differentiated in house.
Mucin: Gastric mucin (ex Sigma Aldrich Inc.)
HBSS buffer: Hank's balanced salt solution buffer (ex Sigma Aldrich Inc.)

### Methods

The volume weighted mean particle size and zeta potential of dispersions were determined by dynamic light scattering (DLS) using a Zeta sizer Nano (Malvern Instruments Ltd, UK) after appropriate dilution. All measurements were carried out at 25°C and the results reported are average of three readings. Diameters are expressed as volume weighted mean particle diameter, unless indicated otherwise. Also the zeta-potential (expressed in millivolt) is determined using this apparatus.

The shape of formed particles was analysed by taking TEM micrographs using a Technai transmission electron microscope (FEI Company, The Netherlands). The dispersion was diluted in MilliQ water and one drop of the diluted dispersion was placed on a 200-mesh carbon-coated copper grid.

Curcumin analysis: UV absorbance was measured at wavelength of 426 nm and the value of absorbance was used to calculate the concentration via the standard calibration curve which was plotted with curcumin concentrations between 2 ppm and 8 ppm beforehand (R=0.9991).

### Example 1: Curcumin - zein particles

### Protocol for colloidal particle preparation using zein

Various zein:curcumin mixtures at certain proportions (50:1 to 5:1 w/w) were dissolved in 100 mL binary solvent ethanol-water (80:20 %v/v) to form a stock solution, followed by addition of these solutions to 300ml demineralised water (MilliQ) under vigorous stirring (1000 rpm) using magnetic stirrer (Model EM3300T, Labotech Inc, Germany). The dispersions thus formed were subjected to solvent removal under reduced pressure using rotary evaporator (Rotavapor R-114, Buchi, Switzerland). The dispersions were then subjected to centrifugation at 4000 rpm for 10 mins to separate out larger aggregates. The final dispersions were then stored at 4°C until further use.

Colloidal dispersions of zein with curcumin with particle size in the range of 90-150 nanometer were prepared, and characterised as indicated in the following table.

**Table 1: Characterisation of prepared particles containing zein and curcumin.**

| | zein:curcumin weight ratio | volume weighted mean particle diameter | zeta potential at pH 4.0 |
|---|---|---|---|
| | [wt:wt] | [nanometer] | [mV] |
| 1.1 | Pure zein particles | 147 | 34.3 |
| 1.2 | 50:1 | 132 | 30.8 |
| 1.3 | 25:1 | 140 | 30.3 |
| 1.4 | 10:1 | 146 | 24.2 |
| 1.5 | 8:1 | 121 | 33.3 |
| 1.6 | 6:1 | 100 | 45.8 |
| 1.7 | 5:1 | 109 | 35.6 |

The TEM micrograph in Figure 1 provides an example of the dense, sheroidal morphology of the zein-curcumin particles, of example 1.7, with a zein:curcumin weight ratio of 5 to 1.

Several experiments were carried out with the colloidal zein-curcumin particle dispersions, as listed below. The experiments reported in this example 1 were conducted with particles having a zein:curcumin weight ratio of 5:1, unless indicated otherwise.

### 1. Enhanced water dispersibility

The aim of the experiment is to see how well formulated curcumin disperses in aqueous media. Unformulated curcumin (pure curcumin) in form of powder and formulated curcumin in form of colloidal dispersion (zein:curcumin weight ratio 5:1) were added to dissolution media and the concentration of curcumin in solution was determined. Curcumin determined included both free as well as complexed or encapsulated curcumin. The difference in the water dispersibility/ solubilisation between zein-curcumin particles and pure curcumin was studied using USP 2 dissolution test. Dissolution media: deionised water with 0.05% Tween 80 wherein equivalent amount of curcumin and curcumin colloidal dispersion were subjected to a release study. Samples were withdrawn at specified time interval (5, 15, 30, 60, 90, 120 and 180 mins) and analysed using UV-vis spectroscopic method.

**Table 2: Comparison of cumulative release vs time for pure curcumin and colloidal dispersion (mean ± standard deviation).**

| time | cumulative release pure curcumin | cumulative release zein-curcumin particles (5:1 wt/wt) |
|---|---|---|
| [min] | [%] | [%] |
| 0 | 0 | 0 |
| 5 | 17.0 ± 0.9 | 99.3 ± 2.0 |
| 15 | 18.1 ± 1.3 | 96.5 ± 4.1 |
| 30 | 19.1 ± 1.4 | 94.2 ± 3.6 |
| 60 | 22.6 ± 1.7 | 91.9 ± 5.4 |
| 90 | 25.7 ± 1.9 | 93.4 ± 4.4 |
| 120 | 27.8 ± 2.0 | 89.8 ± 4.3 |
| 180 | 32.8 ± 2.7 | 92.7 ± 4.1 |

*Results:* Colloidal dispersion showed nearly complete solubilisation with more than 90% release of curcumin in first 5 minutes compared to around 32% for pure curcumin. Curcumin as such is poorly wetted and had limited dissolution which is seen from the concentration of curcumin in solution for unformulated curcumin whereas in case of colloids complete & instant dissolution (more than 90%) of curcumin was obtained.

### 2. pH stability & photostability

Enhanced stability of colloidal dispersion of curcumin-zein to various pH and UV irradiation was studied as follows. A stock solution of pure curcumin was prepared using DMSO and diluted appropriately using buffer at different pH (1.2, 4.5, 6.7, 7.4 and pH 9.0) to get a working concentration of 20 microgram per mL. A colloidal dispersion of zein-curcumin particles (5:1 wt/wt) was also diluted accordingly to achieve a concentration equivalent to 20 microgram per mL of curcumin. Curcumin was quantified in each of these samples after 6 hours. Similarly solutions of curcumin and colloidal dispersions were also subjected to UV irradiation (wavelength 302 nm) using Gel Doc 1000 minitransilluminator (Bio-Rad Laboratories, The Netherlands) for 45 mins.

**Table 3: pH stability of pure curcumin (Cur) and curcumin loaded in colloidal particles (NP).**

| | non-degraded pure curcumin | non-degraded zein-curcumin particles (5:1 wt/wt) |
|---|---|---|
| pH | [%] | [%] |
| 1.2 | 56.0 | 84.3 |
| 4.5 | 69.7 | 74.5 |
| 6.7 | 67.6 | 91.0 |
| 7.4 | 66.6 | 92.1 |
| 9.0 | 52.9 | 88.8 |

*Results:* Curcumin in colloidal dispersion showed better stability at all studied pH as compared to pure curcumin. Zein-curcumin particles were also found to be stable at higher pH 9.0 where it was retained in natural yellow colour as compared to pure ) curcumin, which turned red in colour. It was also noted that the curcumin in colloidal dispersion was most stable at pH 7.4 (physiologically most relevant pH) with less than 10% chemical degradation at the end of 6 hours.

**Table 4: Percent unchanged curcumin level versus time (pure curcumin vs. zein-curcumin particles colloidal dispersion), when irradiated with UV light.**

| | unchanged curcumin level | |
|---|---|---|
| | [%] | |
| time [min] | pure curcumin | zein-curcumin particles (5:1 wt/wt) |
| 0 | 100 | 100 |
| 5 | 93.6 | 99.1 |
| 10 | 88.4 | 88.6 |
| 15 | 66.7 | 78.1 |
| 30 | 59.6 | 78.1 |
| 45 | 47.1 | 69.6 |

Curcumin in colloidal dispersion was also found to be more stable to UV irradiation (302 nm) as compared to pure curcumin. Around 70% of curcumin was found to be unchanged in colloidal dispersion as compared to less than 50% for pure curcumin at the end of 45 minutes, see table 4.

### 3. Stability in digestive conditions

To study the stability of the colloidal dispersion of particles according to the invention in simulated gastro-intestinal conditions, a dissolution test was carried out in simulated gastric and intestinal phases using following conditions. This test indicates whether the particles are suitable to be used as a delivery system for phenolic compounds to the human body.
Zein-curcumin particles (5:1 wt/wt) were used.
For gastric phase: Water with 0.05% Tween , pH 2 in first 2 hours with pepsin
For intestinal phase: pH adjusted to 6.8 and bile salts and pancreatin added to the medium.
Sampling point: (5, 15, 30, 60, 90, 120 and 180 mins). The concentration of curcumin in the dissolution media (combined concentration of dispersed curcumin and curcumin in particles) as was measured over time was used as a measure of stability of the formulation, to see if there was any decrease to the physical instability of curcumin when in colloidal dispersion.

**Table 5: Percent cumulative release versus time for curcumin and colloidal dispersion in both gastric and intestinal phase (mean ± standard deviation).**

| time | release pure curcumin | release zein-curcumin particles (5:1 wt/wt) |
|---|---|---|
| [min] | [%] | [%] |
| 0 | 0 | 0 |
| 5 | 18.0 ± 2.0 | 80.0 ± 5.7 |
| 15 | 18.3 ± 0.3 | 83.7 ± 8.7 |
| 30 | 20.9 ± 2.2 | 81.6 ± 7.8 |
| 60 | 21.7 ± 1.9 | 80.4 ± 6.9 |
| 90 | 38.1 ± 0.5 | 94.6 ± 3.0 |
| 120 | 36.9 ± 1.4 | 94.6 ± 3.8 |
| 150 | 37.6 ± 2.3 | 93.7 ± 4.3 |
| 180 | 37.0 ± 1.2 | 90.2 ± 3.8 |

*Results:* The formulation was found to be stable through out the dissolution test in both gastric and intestinal conditions, the amount of curcumin released not dropping below 80%. This means that only a small amount of curcumin in the particles is degraded. Pure curcumin subjected to similar conditions served as a control, and it was shown that the stability of curcumin in the particles according to the invention was much better than pure curcumin.

### 4. Muco-adhesion properties

The bioadhesive properties of particles according to the invention was determined. Two models were used to determine the bioadhesion, namely an in vitro model and cell studies.
(A) *In vitro* model: agarose was used as muco-mimetic agent, and the bioadhesion was determined by measuring the amount of curcumin eluted by simulated gastric fluid flowing over an agarose gel layer. Procedure was as follows.
A 1.0w/v agarose gel (0.2 cm thick by 2.5 cm long) mounted on a glass slide was placed at an angular elevation of 45° in an incubator (Innova 4080 Incubator Shaker from New Brunswick scientific, The Netherlands) maintained at 37°C. Simulated Gastric Fluid (SGF) comprising of demineralised water (Milli Q) at pH 6.7 at 37°C (temperature maintained using Ecoline 011 Water bath from Lauda, Germany) was made to flow at 1 mL/min over the surface of the agarose gel using a peristaltic pump P-I (Pharmacia Fine Chemicals, Sweden). Pure curcumin suspended in minimum amount of demineralised water (MilliQ) and zein curcumin colloidal dispersion (5:1 wt/wt) was applied directly onto the agarose gel and allowed to dry at ambient temperature. SGF eluent samples (2mL) were collected at time points of 5, 10, 15, 20, 30, 60, 90, 120 and 150 minutes, and analyzed spectrophotometrically.

**Table 6: Percent curcumin retention versus time for colloidal dispersion in in vitro model (mean ± standard deviation).**

| time | curcumin retention zein-curcumin particles (5:1 wt/wt) |
|---|---|
| [min] | [%] |
| 5 | 94.4 ± 0.1 |
| 10 | 91.4 ± 1.3 |
| 20 | 88.2 ± 0.9 |
| 30 | 81.5 ± 4.4 |
| 60 | 70.1 ± 4.0 |
| 90 | 66.2 ± 1.4 |
| 120 | 65.6 ± 1.6 |
| 150 | 65.9 ± 1.6 |

*Results:* Pure curcumin showed no adhesion and was washed off completely in first 5 minutes.The residence time of curcumin was enhanced with more than 60% retention at the end of 150 minutes.
(B) Cell studies: Mucin (the main component of mucosa covering the gastrointestinal lining) was coated onto CaCo2 cells. By addition of phenolic compound in the form of a particle according to the invention, part of the particle will adhere to the mucin and will be transported to the CaCo2 cells. The more compound adheres to the mucin and cells, the better the bioadhesion. The concentration of the compound in the fluid in which the mucin and cells are suspended is determined, and the lower this concentration, the more compound is adhered to the mucin. Concentration of curcumin still in dispersion is measured and after subtracting it from the initial concentration added, the amount of curcumin that bound to mucin can be determined. Hence disappearance of phenolic compound, curcumin in this example, is determined.

CaCo2 cells were differentiated in 12 well plates. Mucin (1% w/v) was prepared in HBSS buffer. 1 ml of above solution was added to each well and incubated for 2 hours for deposition of mucin on the surface of cells. Curcumin, and three different types of particles were added to the cell well and incubated at 37°C. The three particles according to the invention were characterised as:
■ particle A (zein:curcumin ratio 5:1 wt/wt);
■ particle B (zein:curcumin ratio 7.5:1 wt/wt);
■ particle C (zein:curcumin ratio 10:1 wt/wt).

Samples were analysed at 5, 15, 30, 60, 90 and 120 minutes. The concentration of curcumin was analysed spectrophotometrically, and adhesion to the mucin was determined by subtracting the measured concentration from the initial concentration, leading to the adhesion of the curcumin by the mucin.

**Table 7: Curcumin adhesion versus time for curcumin and colloidal dispersions with different zein: curcumin ratios in mucin-CaCo2 cell assay (mean ± standard deviation).**

| | adhesion pure curcumin | adhesion zein-curcumin particles weight ratio zein:curcumin (wt:wt) | | |
|---|---|---|---|---|
| time | | [%] | | |
| [min] | [%] | 5:1 | 7.5:1 | 10:1 |
| 5 | 2.0 ± 0.3 | 50.8 ± 2.1 | 68.0 ± 4.3 | 76.5 ± 3.5 |
| 15 | 8.0 ± 1.0 | 51.8 ± 4.4 | 68.1 ± 2.2 | 81.9 ± 4.0 |
| 30 | 26.6 ± 7.4 | 53.4 ± 2.1 | 70.3 ± 4.0 | 82.9 ± 3.0 |
| 60 | 30.5 ± 2.2 | 59.7 ± 3.7 | 72.5 ± 0.7 | 87.9 ± 2.5 |
| 90 | 37.5 ± 3.3 | 63.0 ± 1.4 | 83.5 ± 1.4 | 88.7 ± 3.5 |
| 120 | 39.0 ± 2.6 | 70.6 ± 3.6 | 83.6 ± 2.0 | 90.5 ± 1.4 |

*Results:* Curcumin adhesion of the particles according to the invention was found to be much higher than that of pure curcumin, and was dependent on zein:curcumin ratio. The adhesion was higher for higher ratios of zein:curcumin signifying the importance of bioadhesive nature of zein.

### Example 2: Theaflavin - zein particles

Similarly as in example 1, particles were prepared containing zein and theaflavin at a ratio of 25:1 (wt/wt). Particle size was determined using a Zeta sizer Nano (Malvern Instruments Ltd, UK), and volume weighted mean diameter was 92.2 nanometer (polydispersity index 0.91, standard deviation of 3 measurements was 0.95 nanometer). The zeta potential was determined to be 41.8 mV at pH 3.8 (standard deviation of 3 measurements was 3.83 mV).

### Example 3: Particles comprising gluten and curcumin, or gluten and theaflavin

Gluten was dispersed in water to get rid of water soluble component (starches, water-soluble protein) followed by dispersion in ethanol:water mixture (80:20 v/v). The soluble fraction was filtered and further used for colloidal particle preparation as described for example 1.

Two types of particles were prepared, gluten with curcumin, and gluten with theaflavins. These were characterised as
■ ratio gluten:curcumin 5:1 wt/wt, mean diameter 314.4 nanometer, standard deviation of 3 measurements of 61.7 nanometer; the zeta potential was determined to be -9.92 mV at pH 5.6 (standard deviation of 3 measurements of 0.53 mV).
■ ratio gluten: theaflavins 25:1 wt/wt, mean diameter 298.5 nanometer, standard deviation of 3 measurements was 8.67 nanometer; the zeta potential was determined to be -13.8 mV at pH 5.2 (standard deviation of 3 measurements of 0.60 mV).

## Claims

1. A composition in the form of a particle comprising a hydrophobic polymer
wherein the polymer comprises one or more prolamines, selected from the group existing of zein, gliadin, hordein, secalin, avenin, and combinations of these prolamines,
and a hydrophobic phenolic compound,
selected from the group existing of curcuminoids, theaflavins, isoflavones, and polymethoxylated flavones, and combinations of these phenolic compounds,
and wherein the particles have a volume weighted mean diameter of between 10 and 1000 nanometer.

2. A composition in the form of a particle according to claim 1, wherein the particles have a volume weighted mean diameter between 80 and 250 nanometer.

3. A composition in the form of a particle according to claim 1 or 2, wherein the particles contain between 1 and 25% by weight of phenolic compound, based on the weight of the particles.

4. A composition in the form of a particle according to any of the previous claims,
wherein the particles contain between 6 and 15% by weight of phenolic compound.

5. A composition in the form of a particle according to any of the previous claims,
wherein the hydrophobic phenolic compound comprises curcumin, and the polymer comprises zein.

6. A composition in the form of a particle according to any of the previous claims,
wherein the particles have a morphology that is substantially convex and compact, preferably ellipsoidal, preferably spheroidal, and preferably substantially spherical.

7. A composition comprising a composition in the form of a particle according to any of claims 1 to 6, wherein the composition is in liquid, solid, or semi-solid form.

8. A composition according to claim 7, wherein the composition is edible.

9. A method for preparation of a composition in the form of a particle according to any of claims 1 to 8, comprising the steps:
a) dissolve hydrophobic polymer and hydrophobic phenolic compound, selected from the group existing of curcuminoids, theaflavins, isoflavones, and polymethoxylated flavones, and combinations of these phenolic compounds, in a mixture of water and organic solvent, wherein the solvent is miscible with water;
b) add the mixture from step a) to water under stirring;
c) separate particles precipitated in step b) from the remaining liquid;
d) optionally dry the particles to remove water and solvent.

10. A method according to claim 9, wherein the mixture of water and organic solvent comprises ethanol, preferably at a ratio of ethanol to water of between 60:40 (v/v) and 90:10 (v/v).

11. A method according to claim 9 or 10, or wherein the hydrophobic phenolic compound comprises curcumin, and the polymer comprises zein.

12. Use according of a composition according to any one of claims 1 to 8 for improving the muco-adhesive properties of hydrophobic phenolic compounds.

## Patentansprüche

1. Zusammensetzung in Form von Teilchen, die Folgendes aufweisen:
ein hydrophobes Polymer, wobei das Polymer ein oder mehrere Prolamine aufweist, die aus der Gruppe ausgewählt sind, die aus Zein, Gliadin, Hordein, Secalin, Avenin und Kombinationen dieser Prolamine besteht,
und eine hydrophobe phenolische Verbindung, die aus der Gruppe ausgewählt ist, die aus Curcuminoiden, Theaflavinen, Isoflavonen und polymethoxylierten Flavonen und Kombinationen dieser phenolischen Verbindungen besteht, und
wobei die Teilchen einen volumengewichteten Mittelwert des Durchmessers von 10 bis 1000 nm aufweisen.

2. Zusammensetzung in Form von Teilchen nach Anspruch 1,
wobei die Teilchen einen volumengewichteten Mittelwert des Durchmessers von 80 bis 250 nm aufweisen.

3. Zusammensetzung in Form von Teilchen nach Anspruch 1 oder 2,
wobei die Teilchen 1 bis 25 Gew.-% phenolische Verbindung enthalten, und zwar auf das Gewicht der Teilchen bezogen.

4. Zusammensetzung in Form von Teilchen nach einem der vorstehenden Ansprüche,
wobei die Teilchen 6 bis 15 Gew.-% phenolische Verbindung enthalten.

5. Zusammensetzung in Form von Teilchen nach einem der vorstehenden Ansprüche,
wobei die hydrophobe phenolische Verbindung Curcumin aufweist und das Polymer Zein aufweist.

6. Zusammensetzung in Form von Teilchen nach einem der vorstehenden Ansprüche,
wobei die Teilchen eine Morphologie aufweisen, die im Wesentlichen konvex und kompakt, vorzugsweise ellipsoid, vorzugsweise sphäroid und vorzugsweise im Wesentlichen sphärisch ist.

7. Zusammensetzung, die eine Zusammensetzung in Form von Teilchen nach einem der Ansprüche 1 bis 6 aufweist, wobei die Zusammensetzung in flüssiger, fester oder halbfester Form vorliegt.

8. Zusammensetzung nach Anspruch 7,
wobei die Zusammensetzung essbar ist.

9. Verfahren zum Herstellen einer Zusammensetzung in Form von Teilchen nach einem der Ansprüche 1 bis 8,
das die folgenden Schritte aufweist:
a) Lösen von hydrophobem Polymer und hydrophober phenolischer Verbindung, die aus der Gruppe ausgewählt ist, die aus Curcuminoiden, Theaflavinen, Isoflavonen und polymethoxylierten Flavonen und Kombinationen dieser phenolischen Verbindungen besteht, in einem Gemisch von Wasser und organischem Lösungsmittel, wobei das Lösungsmittel mit Wasser mischbar ist;
b) Zugeben des Gemischs vom Schritt a) unter Rühren zu Wasser;
c) Abtrennen der im Schritt b) ausgefällten Teilchen von der restlichen Flüssigkeit;
d) gegebenenfalls Trocknen der Teilchen, um Wasser und Lösungsmittel zu entfernen.

10. Verfahren nach Anspruch 9,
wobei das Gemisch von Wasser und organischem Lösungsmittel Ethanol, vorzugsweise in einem Verhältnis von Ethanol zu Wasser von 60:40 (V./V.) bis 90:10 (V./V.) aufweist.

11. Verfahren nach Anspruch 9 oder 10,
wobei die hydrophobe phenolische Verbindung Curcumin aufweist und das Polymer Zein aufweist.

12. Verwendung einer Zusammensetzung nach einem der Ansprüche 1 bis 8 für die Verbesserung der Schleimhauthaftungseigenschaften von hydrophoben phenolischen Verbindungen.

## Revendications

1. Composition sous la forme d'une particule comprenant un polymère hydrophobe, dans laquelle le polymère comprend une ou plusieurs prolamines choisies dans le groupe constitué de zéine, de gliadine, d'hordéine, de sécaline, d'avénine et des combinaisons de ces prolamines,
et un composé phénolique hydrophobe,
choisi dans le groupe constitué de curcuminoïdes, théaflavines, isoflavones et flavones polyméthoxylées et les combinaisons de ces composés phénoliques et dans laquelle les particules ont un diamètre volumique moyen pondéré entre 10 et 1000 nanomètres.

2. Composition sous la forme d'une particule selon la revendication 1, dans laquelle les particules ont un diamètre volumique moyen pondéré entre 80 et 250 nanomètres.

3. Composition sous la forme d'une particule selon la revendication 1 ou 2, dans laquelle les particules contiennent entre 1 et 25 % en poids de composé phénolique sur la base du poids de la particule.

4. Composition sous la forme d'une particule selon l'une des revendications précédentes, dans laquelle les particules contiennent entre 6 et 15 % en poids de composé phénolique.

5. Composition sous la forme d'une particule selon l'une quelconque des revendications précédentes, dans laquelle le composé phénolique hydrophobe comprend de la curcumine et le polymère comprend de la zéine.

6. Composition sous la forme d'une particule selon l'une quelconque des revendications précédentes, dans laquelle les particules ont une morphologie qui est substantiellement convexe et compacte, de préférence, ellipsoïde, de préférence, sphéroïde et de préférence, substantiellement sphérique.

7. Composition comprenant une composition sous la forme d'une particule selon l'une quelconque des revendications 1 à 6, dans laquelle la composition est sous une forme liquide, solide ou semi-solide.

8. Composition selon la revendication 7, la composition étant comestible.

9. Méthode pour la préparation d'une composition sous la forme d'une particule selon l'une quelconque des revendications 1 à 8, comprenant les étapes de :
a) dissolution du polymère hydrophobe et du composé phénolique hydrophobe, choisi dans le groupe constitué de curcuminoïdes, de théaflavines, d'isoflavones et de flavones polyméthoxylées et des combinaisons de ces composés phénoliques, dans un mélange d'eau et de solvant organique, dans lequel le solvant est miscible avec de l'eau ;
b) addition du mélange de l'étape a) à de l'eau sous agitation ;
c) séparation des particules précipitées à l'étape b) du liquide restant ;
d) éventuellement, séchage des particules pour éliminer l'eau et le solvant.

10. Méthode selon la revendication 9, dans laquelle le mélange d'eau et de solvant organique comprend l'éthanol, de préférence en un rapport d'éthanol à l'eau entre 60 : 40 (v/v) et 90 : 10 (v/v).

11. Méthode selon la revendication 9 ou 10, ou dans laquelle le composé phénolique hydrophobe comprend de la curcumine, et le polymère comprend de la zéine.

12. Utilisation d'une composition selon l'une quelconque des revendications 1 à 8, pour améliorer les propriétés muco-adhésives des composés phénoliques hydrophobes.
